# EUROPEAN PATENT SPECIFICATION

(11) **EP 0 883 403 B1**
(45) Date of publication and mention of the grant of the patent: **25.08.2010**
(21) Application number: 97903127.5
(22) Date of filing: 28.01.1997
(51) Int. Cl.: A61K 31/54, A61K 31/495, A61K 31/50, A61K 31/36, A61K 31/445, A61P 15/10

(54) **METHOD FOR TREATING SEXUAL DYSFUNCTIONS**
METHODE ZUR BEHANDLUNG SEXUELLER FEHLFUNKTIONEN
METHODE DE TRAITEMENT DE DYSFONCTIONNEMENTS SEXUELS

(30) Priority: 29.01.1996 US 10778 P
(43) Date of publication of application: 16.12.1998
(73) Proprietor: The Regents of The University of California, Oakland, California 94612-3550 (US)
(72) Inventor: GRANGER, Richard, H., Irvine, CA 92715 (US); LYNCH, Gary, S., Irvine, CA 92715 (US)
(74) Representative: Bradley, Adrian
(86) International application number: PCT/US1997/001285
(87) International publication number: WO 1997/026884

(56) References cited:
- WO-A-94/02475
- US-A- 4 022 893
- MELIS M R ET AL: "Penile erection and yawning induced by paraventricular NMDA injection in male rats are mediated by oxytocin." PHARMACOLOGY BIOCHEMISTRY AND BEHAVIOR, vol. 48, no. 1, 1994, pages 203-207, XP001013456 ISSN: 0091-3057
- LOUIS J. IGNARRO: "Nitric oxide as the physiological mediator of penile erection" JOURNAL OF NIH RESEARCH, vol. 4, May 1992 (1992-05), pages 59-62, XP001017840
- ARGIOLAS: "Nitric oxide is a central mediator of penile erection" NEUROPHARMACOLOGY, vol. 33, no. 11, 1994, pages 1339-1344, XP001012834
- BHARDWAJ A ET AL: "In-vivo characterization of nitric oxide (NO) production in the hippocampus with N-methyl-D-aspartate (NMDA) and alpha-amino-3-hydroxy-5-methylisoxazole-4- propionic acid (AMPA) receptor stimulation and blockade." SOCIETY FOR NEUROSCIENCE ABSTRACTS, vol. 20, no. 1-2, 1994, page 650 XP001013495 24th Annual Meeting of the Society for Neuroscience;Miami Beach, Florida, USA; November 13-18, 1994 ISSN: 0190-5295

## Description

The present invention relates to compositions for treating sexual dysfunctions in mammalian subjects, particularly human males wherein the sexual dysfunctions are neurogenic, psychogenic or age-related.

The DSM-IIIR defines four phases of sexual response (appetite, excitement, orgasm, resolution). Inhibition of any of these is collectively known as "sexual dysfunction." Sexual dysfunction in males specifically encompasses the inability to sustain a penile erection (Male Erectile Disorder or impotence), the inability to ejaculate and/or the inability to experience orgasm.

According to the DSM-III-R (p. 292), approximately 8% of young adult males have Male Erectile Disorder. An estimated 20% of the total population have Hypoactive Sexual Desire Disorder.

Male sexual dysfunction may be due to one or more causes.

About forty to sixty percent of cases of Male Erectile Disorder is associated with atherosclerosis of the arteries supplying blood to the penis ("arteriogenic" or "atherosclerotic" dysfunction). In some cases, there is leakage from veins in the penis such that sufficient pressure for an erection can neither be obtained nor maintained.

"Neurogenic" sexual dysfunction associated with a neuropathy (*e.g*., nerve damage in the nervous system affecting the penis arising from, for example, surgery or a pelvic injury) accounts for about ten to fifteen percent of impotence cases.

Psychological or "psychogenic" dysfunction, resulting, for example, from anxiety or depression, with no apparent substantial somatic or organic impairment, accounts for about fifteen to twenty percent of cases of impotence. These include an age-associated sexual decline which occurs unaccompanied by any overt pathologies.

Erectile insufficiency is sometimes a side effect of certain drugs, such as beta-blockers that are administered to reduce blood pressure in persons suffering from hypertension, or drugs administered to treat depression or anxiety. Excessive alcohol consumption is a cause of erectile insufficiency. These forms of sexual dysfunction may be regarded as a subset of neurogenic or psychogenic insufficiency.

In most cases, no hormonal or primarily structural (anatomical) defects are clinically demonstrable. This common type of impotence usually increases in frequency with advancing age. About twelve percent of adult men under age forty-five, about twenty percent of men at age sixty, and about fifty-five percent of men at age seventy-five experience difficulty in engaging in sexual intercourse.

A number of methods to treat sexual dysfunction are available. These treatments include surgery (*e.g*., penile implants), pharmacological treatments and, in cases of psychogenic dysfunction, psychological counseling. See, *e.g*., U.S. Patent No. 5,399,581 to Laragh, Mar. 21, U.S. Patent No. 5,439,007 to Fischer, Aug. 8, 1995, U.S. Patent No. 5,447,912 to Gerstenberg et al., Sep. 5, 1995, U.S. Patent No. 5,451,609 to Bellamy, Sep. 19, 1995, and references cited therein.

These published pharmacological methods of treatment have primarily targeted peripheral nervous system responses. They have had only limited success, and there are potentially severe side-effects.

Testosterone has been shown to be effective in modulating sexual behavior in a variety of mammals, including rats and humans. See, *e.g*., Smith et al. (1992), Hormones and Behaviour 26: 110-135 (rats) and Carani et al. (1990), Arch. Sex. Behav. 19: 223-234 (humans). However, steroid treatments have the potential for eliciting undesirable behaviors, for example, aggression.

Injection of or moxisylyte (ICAVEX) (U.S. Patent No. 5,451,609 to Bellamy, Sep. 19, 1995), the smooth muscle relaxant papaverine, or the non-specific blocker and hypotensive phenoxybenzamine into a corpus cavernosum has been reported to cause an erection sufficient for vaginal penetration. Papaverine is effective in cases where the dysfunction is psychogenic or neurogenic and severe atherosclerosis is not involved.

In cases where severe atherosclerosis is not a cause of the dysfunction, intracavernosal injection of phentolamine, an alpha-adrenergic blocker, causes an erection that suffices for vaginal penetration. The resulting erection is one of significantly shorter duration than that induced by intracavernosal injection of papaverine or phenoxybenzamine. It often is of such short duration that satisfactory sexual relations are difficult or impossible.

The neuropeptide vasoactive intestinal peptide ("VIP") is thought to be associated with erections in normal males (i.e., males not suffering from erectile insufficiency). Injection of VIP into a corpus cavernosum of a normal male without subjecting the male to sexual stimulation causes slight swelling (slight tumescence) of the penis but not an erection. When coupled with visual sexual stimulation or sexual stimulation by vibration or both, injection of small amounts of VIP into a corpus cavernosum of a normal male facilitates full erection (see, Wagner et al. (1987) World J. Urol. 5: 171-172). VIP alone or coupled with sexual stimulation does not induce erections in males whose impotence is due to severe atherosclerosis.

It will be readily apparent that such invasive treatments require of the patient a great sense of purpose and a single-minded dedication to the task at hand, and that they may actually hinder rather than facilitate sexual activity. Intracavernosal injection is often associated with a burning sensation after the injection. In addition, papaverine or phenoxybenzamine can result in priapism, a persistent erection lasting from a few hours to sometimes longer than twenty-four hours. Priapism is a serious side effect of treatment of erectile insufficiency with these drugs. Beyond the embarrassment that some men may experience, priapism is often painful, can irreversibly damage erectile tissue, and, to be relieved, requires bleeding or pharmacological intervention, such as injection of a sympathomimetic drug such as adrenaline. There are indications that repeated use of papaverine causes extensive intracavernous fibrosis. MELIS M R ET AL ("Penile erection and yawning induced by paraventricular NMDA injection in male rats are mediated by oxytocin." PHARMACOLOGY BIOCHEMISTRY AND BEHAVIOR, vol. 48, no. 1, 1994, pages 203-207) and ARGIOLAS ("Nitric oxide is a central mediator of penile erection" NEUROPHARMACOLOGY, vol. 33, no. 11, 1994, pages 1339-1344) fail to mention the compounds of Formula I or AMPA upmodilators as described in the present specification.

In summary, although sexual dysfunction is a pervasive problem, there are few satisfactory compositions available for treating this disorder. Therefore, it is an object herein to provide compositions for treating sexual dysfunctions as described in claim 1.

### SUMMARY OF THE INVENTION

The present invention is based on the discovery that male sexual dysfunction can be treated by compounds that enhance glutamatergic neural transmission. The invention thus comprehends the use of a compound having the following formula (with ring vertices numbered as shown): 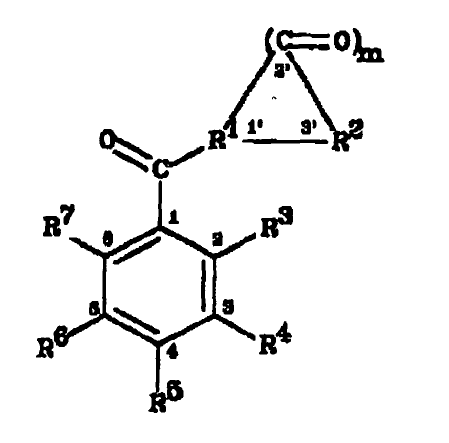 in which:
R¹ is a member selected from the group consisting of N and CH;
m is O or 1;
R² is (CHR⁸)ₙ₋ₘ or Cₙ₋ₘHR⁸₂₍ₙ₋ₘ₎₋₃,
in which n is 4, 5, 6, or 7, the R⁸'s in any single compound being the same or different, each R⁸ being a member selected from the group consisting of H and C₁-C₆ alkyl;
R³ is a member selected from the group consisting of H, C₁-C₆ alkyl, and C₁-C₆ alkoxy;
R⁶ is H;
R⁴ and R⁵ are combined to form a member selected from the group consisting of 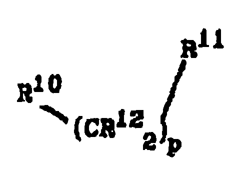 and 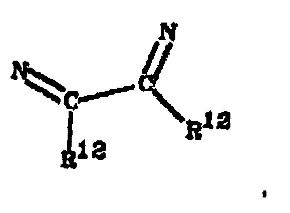 in which:
R¹⁰ and R¹¹ are O;
R¹² is a member selected from the group consisting of H and CH₃;
p is 1 or 2; and
R⁷ is a member selected from the group consisting of H, C₁-C₆ alkyl, and C₁-C₆ alkoxy, wherein said compound is capable of upmodulating the response of α-amino-3-hydroxy-5-methyl-isoxazole-4-propionic acid ("AMPA") receptors to natural ligand binding, in the manufacture of a medicament for the treatment of the claimed sexual dysfunctions.

A number of glutamatergic up-modulators may be used in the present invention; for example, 7-chloro-3-methyl-3-4-dihydro-2H-1,2,4 benzothiadiazine S,S, dioxide.

Typical dosages for systemic administration can range from milligrams to decigrams per kg weight of subject per administration. Preferably, the glutamatergic drug used has a rapid onset and short half-life (≤ 90 min.).

The medicament can be used for treating a sexual dysfunction that is substantially psychogenic in origin. Preferentially, the human patient is one that has a reduced sexual drive, arousal or desire, age-associated hyposexuality, unaccompanied by other pathological anatomical indications or difficulties in reaching erection and/or orgasm. The compound used in accordance with the invention may reduce intromission latency and ejaculatory latency and promote erections sufficient for vaginal penetration in male mammals suffering from sexual dysfunctions that are predominantly psychogenic in nature. However, said compounds may also be effective to treat psychogenic symptoms in individuals whose sexual disfunction is partly neurogenic or due to peripheral pathologies. Thus, said medicament may be administered to the male, and thereafter the male is sexually stimulated.

Said medicament may be provided in the form of tablets or ampules or other suitable packaging means, formulated for controlled dosage administration.

### BRIEF DESCRIPTION OF THE DRAWINGS

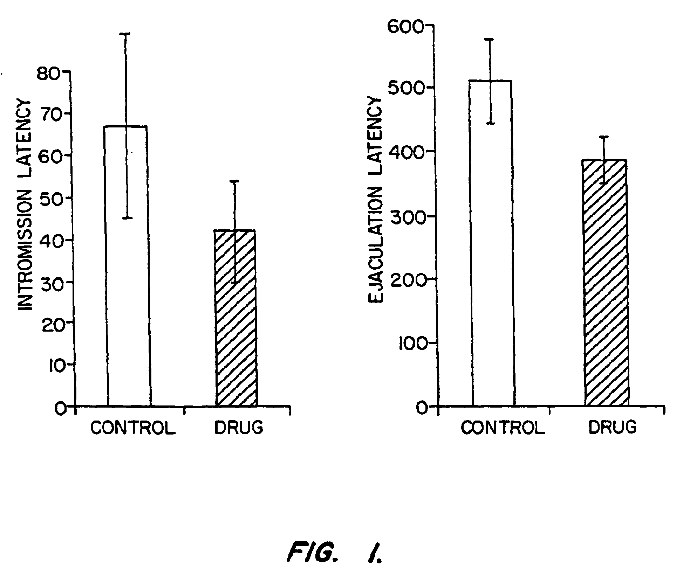

Fig. 1 compares intromission and ejaculatory latency in aged rats treated with glutamatergic enhancers, as compared to control rats.

### DETAILED DESCRIPTION OF THE INVENTION

Sexual behaviour generally comprises arousal (excitement), courtship displays, erotic manipulation, intercourse, orgasm and resolution. All of these are affected by neural and hormonal circuitries. The neural circuits involve central neural system and peripheral nervous system components.

Two principal elements of the male repertoire of sexual activity are erection and ejaculation. The first element, erection, is thought to be mediated by the parasympathetic nervous system. Cholinergic impulses from the spinal cord cause arterial dilatation and venous constriction in the penis. The arterial blood in the erectile tissue of the penis builds up under high pressure due to the venous constriction which blocks the outflow of blood from the penis. The second element, ejaculation, is thought to be mediated by the sympathetic nervous system. Sensory nerves in the penis respond to sexual stimulation by sending sensory impulses to the spinal cord. After processing these impulses, the spinal cord sends sympathetic impulses to the male reproductive organs, causing them to contract. This contraction results in the filling of the internal urethra with sperm and prostatic fluid. The corresponding pressure increase in the internal urethra stimulates pressure receptors which send impulses to the spinal cord. Cholinergic impulses are then sent from the spinal cord to the penis where the skeletal muscle surrounding the erectile tissue is stimulated to contract, causing ejaculation of the sperm from the internal urethra.

Existing approaches to treat male sexual dysfunction typically involve hormones and lower brain function. Much recent activity in sexual therapy has been aimed at the peripheral nervous system ("PNS"), largely because these systems are better understood. These approaches have yielded limited clinical success.

There is evidence that there is significant central nervous system ("CNS") participation in most instances of sexual performance deficits. For example, involvement of the striatal complex (nucleus accumbens septi) and the frontal cortex has been reported in sexual behavior (Robertson *et al.,* 1991).

Most excitatory neurotransmission in these and other forebrain structures occurs via glutamate. The majority of CNS glutamatergic receptors are of the AMPA class. The remaining are of the NMDA class. *See*, Jonas, P., 1993, Exs, 66:61-76; Zorumsky et al., 1993, Pharmacology and Therapeutics, 59:145-162; and Orrego et al., 1993, Neuroscience, 56:539-555.

Facilitation of central (brain) glutamatergic transmission via up-modulation of "AMPA-type" glutamate receptors has larger effects on complex, polysynaptic circuitries than on simple circuits (Sirvio *et al.,* 1995), and hence is expected to produce greater facilitation of the types of network functions found in neocortex than those located in the lower brain. From this perspective, centrally active up-modulators of the AMPA receptors should enhance the cortical component of elaborate behaviors, including sexual interactions, while minimally altering lower brain visceral activities.

The glutamatergic enhancers of this invention have as one target CNS pathways that underlie both sexual interest (libido, desire) and sexual function (arousal, copulation, orgasm). In brief, stimulated presynaptic neurons release neurotransmitters such as glutamate into the synaptic cleft. The neurotransmitter binds to receptors present on the postsynaptic neuron. The formation of the ligand-receptor complex results in the opening of ion channels which permit the passage of ions across the neural plasma membrane of the post-synaptic neuron. If the net flux of ions exceeds a certain threshold level, the postsynaptic neuron is stimulated.

The compounds used in the present invention primarily act, not by directly stimulating neural activation, but by upmodulating ("allosteric modulation") neural activation and transmission in neurons that contain glutamatergic receptors. These compounds bind to the glutamate receptor at a site other than the glutamate binding site, but such binding does not by itself give rise to ion fluxes. However, when a glutamate molecule binds to a glutamate receptor that has bound to it a glutamatergic compound of the invention, the subsequent ion flux is of much longer duration. Thus, in the presence of the compounds used herein, postsynaptic neurons are activated by much lower concentrations of glutamate than postsynaptic neurons that do not contain bound compounds.

### A. Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described. For purposes of the present invention, the following terms are defined below.

"Cyano" refers to the group -CN.

"Halogen" or "halo" refers to fluorine, bromine, chlorine, and iodine atoms.

"Hydroxy" refers to the group -OH.

"Thiol" or "mercapto" refers to the group -SH.

"Sulfamoyl" refers to the -SO₂NH₂.

"Alkyl" refers to a cyclic, branched or straight chain, alkyl group of one to eight carbon atoms. The term "alkyl" includes reference to unsubstituted alkyl groups. This term is further exemplified by such groups as methyl, ethyl, n-propyl, i-propyl, n-butyl, t-butyl, i-butyl (or 2-methylpropyl), cyclopropylmethyl, cyclohexyl, i-amyl, n-amyl, and hexyl. "Lower alkyl" refers to C₁-C₆ alkyl, with C₁-C₄ alkyl more preferred. "Cyclic alkyl" includes both mono-cyclic alkyls, such as cyclohexyl, and bi-cyclic alkyls, such as [3.3.0]bicyclooctane and [2.2.1]bicycloheptane.

"Aryl" or "Ar" refers to an aromatic substituent which may be a single ring or multiple rings which are fused together, linked covalently, or linked to a common group such as an ethylene or methylene moiety. The aromatic ring(s) may contain a heteroatom, such as phenyl, naphthyl, biphenyl, diphenylmethyl, 2,2-diphenyl-1-ethyl, thienyl, pyridyl and quinoxalyl. The term "aryl" or "Ar" includes reference to both substituted and unsubstituted aryl groups. If substituted, the aryl group may be substituted with halogen atoms, or other groups such as hydroxy, cyano, nitro, carboxyl, alkoxy, phenoxy, fluoroalkyl and the like. Additionally, the aryl group may be attached to other moieties at any position on the aryl radical which would otherwise be occupied by a hydrogen atom (such as 2-pyridyl, 3-pyridyl and 4-pyridyl).

The term "alkoxy" denotes the group -OR, where R is lower alkyl, substituted lower alkyl, aryl, substituted aryl, aralkyl or substituted aralkyl as defined below.

The term "acyl" denotes groups -C(O)R, where R is alkyl, substituted alkyl, alkoxy, aryl, substituted aryl, amino and alkylthiol.

"Carbocyclic moiety" denotes a ring structure in which all ring vertices are carbon atoms. The term encompasses both single ring structures and fused ring structures. Examples of aromatic carbocyclic moieties are phenyl and naphthyl.

"Heterocyclic moiety" denotes a ring structure in which one or more ring vertices are atoms other than carbon atoms, the remainder being carbon atoms. Examples of non-carbon atoms are N, O, and S. The term encompasses both single ring structures and fused ring structures. Examples of aromatic heterocyclic moieties are pyridyl, pyrazinyl, pyrimidinyl, quinazolyl, isoquinazolyl, benzofuryl, isobenzofuryl, benzothiofuryl, indolyl, and indolizinyl.

The term "amino" denotes the group NRR', where R and R' may independently be hydrogen, lower alkyl, substituted lower alkyl, aryl, substituted aryl as defined below or acyl.

The term "amido" denotes the group -C(O)NRR', where R and R' may independently be hydrogen, lower alkyl, substituted lower alkyl, aryl, substituted aryl as defined below or acyl.

The term "independently selected" is used herein to indicate that the two R groups, R¹ and R², may be identical or different (*e.g*., both R¹ and R² may be halogen or, R¹ may be halogen and R² may be hydrogen, *etc*.).

A "sexual dysfunction" generally denotes the inhibition of any one or more of the phases of sexual response (appetite, excitement, orgasm, resolution) described in the DSM-IIIR. "Sexual dysfunction" specifically encompasses decreased sexual desire (Hypoactive Sexual Desire Disorder, DSM-III-R # 302.71), the inability to sustain a penile erection (Male Erectile Disorder, DSM-III-R # 302.72), the inability to ejaculate and/or the inability to experience orgasm (Inhibited Male Orgasm, DSM-III-R # 302.74). All may be psychogenic only, or psychogenic and biogenic, lifelong or acquired, and generalized or situational. The DSM-IIIR definitions and text relating to sexual dysfunction are hereby incorporated by reference.

"Impotence" or "Male Erectile Disorder" is the consistent inability to maintain an erect penis of sufficient rigidity for sexual intercourse.

"Sexual behavior" may or may not involve a partner. Where a partner is involved, sexual behavior comprises arousal, courtship displays and copulation. Arousal (or excitement) consists of a subjective sense of sexual pleasure and accompanying physiological changes. The major changes in the male comprise penile tumescence leading to erection. Courtship displays are behaviors intended to or having the effect of arousing a sexual partner and of increasing the arousal of the actor. Copulation may comprise intromission of the penis into the sexual partner's sexual organs (in heterosexual copulation), orgasm and ejaculation. Where a partner is not involved, sexual behavior may include any combination of touching or erotically manipulating erogenous areas of the genital organs or other erogenous parts of the body (*e.g*., masturbation); responding to visual stimulation such as pictorial depiction of erotic acts and objects.

The phrase "symptoms of sexual dysfunction" includes inhibition of any of the four phases of sexual response (appetite, excitement, orgasm, resolution) mentioned in the DSM-IIIR. These specifically include lack of sexual desire (Hypoactive Sexual Desire Disorder, DSM-III-R # 302.71), impotence or the inability to sustain a penile erection (Male Erectile Disorder, DSM-III-R # 302.72), the inability to ejaculate and/or the inability to experience orgasm (Inhibited Male Orgasm, DSM-III-R # 302.74).

The phrase "diminish the symptoms of sexual dysfunction" denotes a decrease in the inhibition of any one or more of the four phases of sexual response (appetite, excitement, orgasm, resolution) described in the DSM-IIIR. The phrase specifically encompasses increased sexual desire, the enhanced ability to sustain a penile erection, the enhanced ability to ejaculate and/or to experience orgasm. A particular example of diminished symptoms of sexual dysfunction is an increase in the number, frequency and duration of instances of sexual behavior or of subjective sexual arousal.

The term "subject" means a male mammal, particularly a human. The term specifically includes domestic and common laboratory mammals, such as non-human primates, kine, horses, pigs, goats, sheep, rabbits, rats and mice.

"Age-related sexual dysfunctions" are sexual dysfunctions that are manifested in aging subjects and that often worsen with increasing age. They are common to both human and animal species (Davidson *et al.,* 1983a; 1983b; Smith and Davidson, 1990).

"α-amino-3-hydroxy-5-methyl-isoxazole-4-propionic acid", or "AMPA", or "glutamatergic" receptors are molecules or complexes of molecules present in cells, particularly neurons, usually at their surface membrane, that recognize and bind to glutamate or AMPA. The binding of AMPA or glutamate to an AMPA receptor normally gives rise to a series of molecular events or reactions that result in a biological response. The biological response may be the activation or potentiation of a nervous impulse, changes in cellular secretion or metabolism, or causing cells to undergo differentiation or movement.

The term "central nervous system" or "CNS" comprises the brain and the spinal cord. The term "peripheral nervous system" or "PNS" comprises all parts of the nervous system that are not part of the CNS, including cranial and spinal nerves and the autonomic nervous system.

The phrase "effective amount" means a dosage sufficient to produce a desired result. Generally, the desired result is a subjective or objective decrease in the symptoms of sexual dysfunction, as measured by the techniques described below.

### B. Compounds used to treat sexual dysfunction.

Compounds useful in the practice of this invention are generally those which amplify (upmodulate) the activity of the natural stimulators of AMPA receptors, particularly by amplifying excitatory synaptic response. We describe herein a wide variety of diverse compounds suitable for use in the invention. Methods for identifying other compounds are routine. They involve a variety of accepted tests to determine whether a given candidate compound is an upmodulator of the AMPA receptor. The primary assay is measurement of enlargement of the excitatory postsynaptic potential (EPSP) in *in vitro* brain slices, such as rat hippocampal brain slices.

In experiments of this kind, slices of hippocampus from a mammal such as rat are prepared and maintained in an interface chamber using conventional methods. Field EPSPs are recorded in the stratum radiatum of region CA1b and elicited by single stimulation pulses delivered once per 20 seconds to a bipolar electrode positioned in the Schaffer-commissural projections (see Granger, R. et al., 1993, Synapse, 15:326-329; Staubli, U. et al., 1994a, Proc. Nat. Acad. Sci., 91:777-781; and Staubli, V. et al., 1994b, Proc. Nat. Acad. Sci., 91:11158-11162; Arai, A. et al., 1994, Brain Res. , 638:343-346; Arai, A. *et al.,* "Effects of a centrally active drug on AMPA receptor kinetics).

The wave form of a normal EPSP is composed of:
an AMPA component, which has a relatively rapid rise time in the depolarizing direction (~5-10 msec) and which decays within ~ 20 msec.;
an NMDA component (slow ~ 30-40 msec rise time and slow ~ 40-70 msec decay) (the NMDA portion will not appear in normal CSF media, due to the voltage requirement for NMDA receptor channel activation, but in low magnesium media, an NMDA component may appear;
a GABA component in the opposite (hyperpolarizing) direction as the glutamatergic (AMPA and NMDA) components, exhibiting a time course with a rise time of ~ 10-20 msec and very slow decay (~50-100 msec or more).

The different components can be separately measured to assay the effect of a putative AMPA receptor enhancing agent. This is accomplished by adding agents that block the unwanted components, so that the detectable responses are essentially only AMPA responses. For example, to measure AMPA responses, an NMDA receptor blocker (e.g., AP-5 or other NMDA blockers known in the art) and/or a GABA blocker (e.g., picrotoxin or other GABA blockers known in the art) are added to the slice. To prevent epileptiform activity in the GABA-blocked slices, known agents such as tetrodotoxin may be used.

AMPA upmodulators useful in the present invention are substances that cause an increased ion flux through the AMPA receptor complex channels in response to glutamatergic stimulation. Increased ion flux is typically measured as one or more of the following non-limiting parameters: at least a 10% increase in decay time, amplitude of the waveform and/or the area under the curve of the waveform and/or a decrease of at least 10% in rise time of the waveform, for example in preparations treated to block NMDA and GABA components. The increase or decrease is preferably at least 25-50%; most preferably it is at least 100%. How the increased ion flux is accomplished (e.g., increased amplitude or increased decay time) is of secondary importance; upmodulation is reflective of increased ion fluxes through the AMPA channels, however achieved.

An additional and more detailed assay is that of excised patches, i.e., membrane patches excised from cultured hippocampal slices; methods are described in Arai *et al.,* 1994. Outside-out patches are obtained from pyramidal hippocampal neurons and transferred to a recording chamber. Glutamate pulses are applied and data are collected with a patch clamp amplifier and digitized (Arai *et al.,* 1994).

Because these membrane patches should contain only glutamatergic receptors, GABAergic currents will not be seen. Any NMDA currents can be blocked as above (e.g., with AP-5).

The central action of a drug can be verified by measurement of Field EPSPs in behaving animals (see Staubli *et al.,* 1994a) and time course of biodistribution can be ascertained via injection and PET measurement of radiolabeled drug (see Staubli *et al.,* 1994b).

One such class of compounds is defined by Formula I:

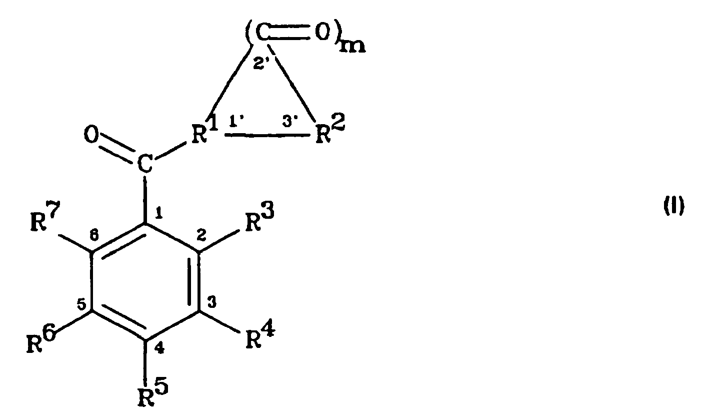

In this formula:
R¹ is either N or CH;
m is either O or 1;
R² is (CHR⁸)ₙ₋ₘ or Cₙ₋ₘHR⁸₂₍ₙ₋ₘ₎₋₃, in which:
   n is 4, 5, 6, or 7; and
   the R⁸'s in any single compound are either the same or different, each R⁸ being either H or C₁-C₆, alkyl;
R³ is either H, C₁-C₈ alkyl, or C₁-C₆ alkoxy;
R⁶ is H;
R⁴ and R⁵ are combined to form a member selected from the group consisting of 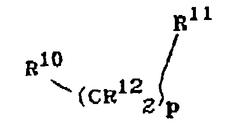 and 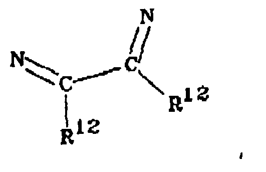 in which:
   R¹⁰ and R¹¹ are O; R¹² is H or CH₃;
   p is 1, or 2; and
R⁷ is either H, C₁-C₆ alkyl, or C₁-C₆ alkoxy.

Within the scope of Formula I, certain subclasses are preferred. One of these is the subclass in which R² is (CHR⁸)ₙ₋ₘ, or Cₙ₋ₘHR⁸₂₍ₙ₋ₘ₎₋₃, and R³ is H, C₁-C₆ alkyl, or C₁-C₆ alkoxy.

When R⁴ and R⁵ are combined, a preferred subclass for R¹² is H and CH₃, and preferred groups representing the combination of R⁴ and R⁵ are 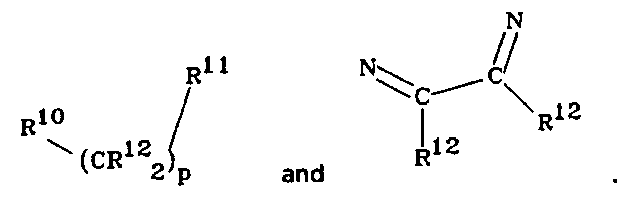 In these groups, R¹⁰ and R¹¹ are both preferably O and p is 1 or 2.

Compounds 1 through 25 below are examples of compounds within the scope of Formula I:

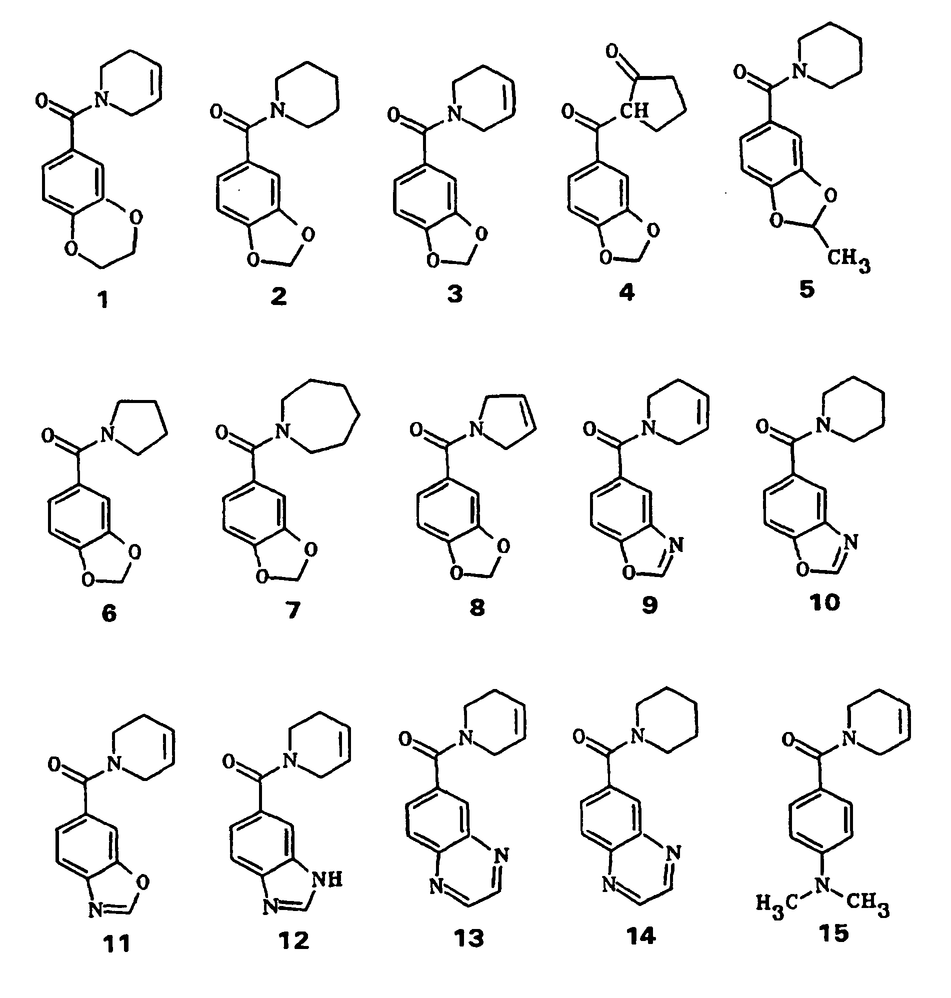

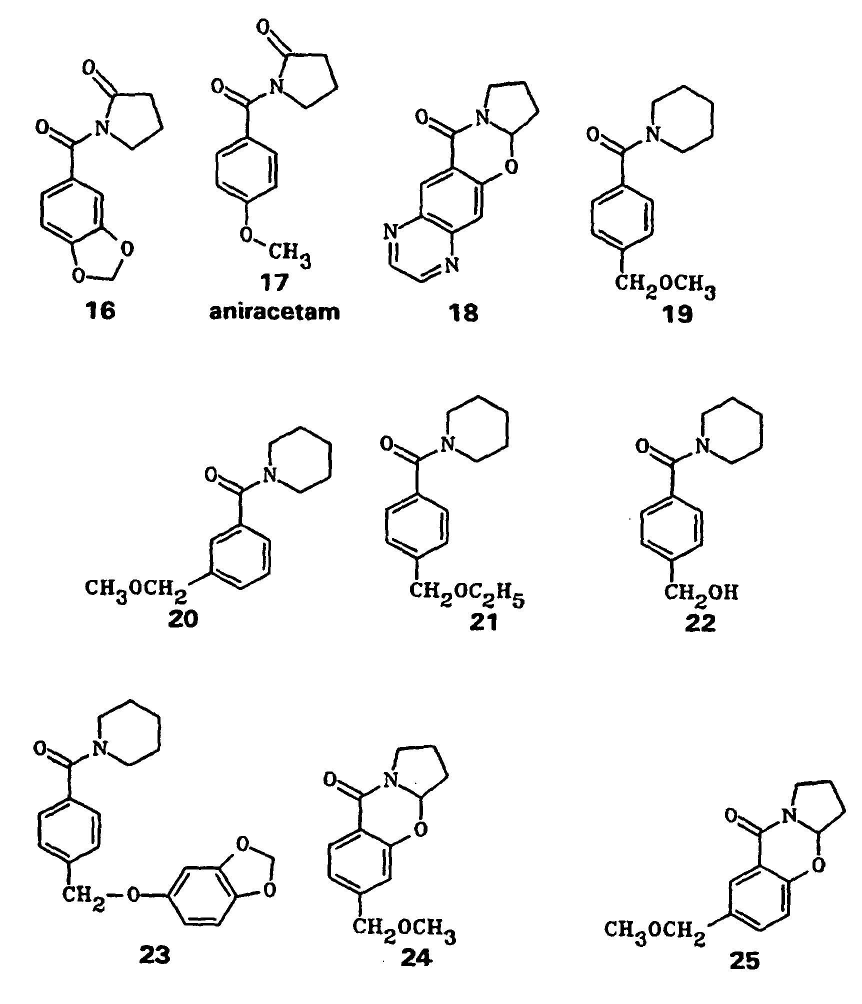

A particularly preferred compound is compound CX 516, 1-(Quinoxalin-6-ylcarbonyl)piperidine, having the following structure:

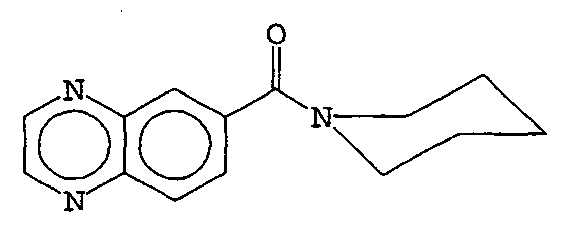

### 1. Preparation of Formula I compounds

The compounds described above can be prepared by conventional methods known to those skilled in the art of synthetic organic chemistry. For example, certain compounds of Formula I can be prepared from an appropriately substituted benzoic acid by contacting the acid under conditions suitable to activate the carboxy group for the formation of an amide. This is accomplished, for example, by activating the acid with carbonyl diimidazole, or with a chlorinating agent such as thionyl chloride or oxalyl chloride to obtain the corresponding benzoyl chloride. The activated acid is then contacted with a nitrogen-containing heterocyclic compound under conditions suitable for producing the desired imide or amide. Alternatively, the substituted benzoic acid can be ionized by contact with at least two equivalents of base such as triethylamine in an inert solvent such as methylene chloride or alcohol-free chloroform, and the ionized benzoic acid can then be reacted with pivaloyl chloride or a reactive carboxylic acid anhydride such as trifluoroacetic anhydride or trichloroacetic anhydride, to produce a mixed anhydride. The mixed anhydride is then contacted with a nitrogen-containing heterocyclic compound to produce the desired imide or amide.

A further alternative to these methods, suitable for some of the compounds of Formula I, is to contact the appropriately selected 3,4-(alkylenedihetero)-benzaldehyde with ammonia to form an imine, then contacting the imine with benzoyloxycarbonyl chloride to form the benzoyloxycarbonyl imine. Suitable 3,4-(alkylenedihetero)-benzaldehydes include 3,4-(methylenedioxy)-benzaldehyde, 3,4-(ethylenedioxy)-benzaldehyde, 3,4-(propylenedioxy)-benzaldehyde, 3,4-(ethylidenedioxy)-benzaldehyde, 3,4-(propylenedithio)-benzaldehyde, 3,4-(ethylidenedithio)-benzaldehyde, 5-benzimidazolecarboxaldehyde, and 6-quinoxalinecarboxaldehyde. The benzoyloxycarbonyl imine is then contacted with a simple conjugated diene such as butadiene under cycloaddition reaction conditions, and then with a Lewis acid under conditions suitable for a Friedel-Crafts acylation. Examples of suitable conjugated dienes include butadiene, 1,3-pentadiene, and isoprene, and examples of suitable Lewis acids include AlCl₃ and ZnCl₂.

Still further compounds within Formula I are prepared from 2,3-dihydroxy naphthalene. This starting material is reacted with 1,2-dibromoethane in the presence of base to produce an ethylenedioxy derivative of naphthalene, which is then reacted with an oxidizing agent such as potassium permanganate to produce 4,5-ethylenedioxyphthaldehydic acid. The latter is contacted with anhydrous ammonia to form an imine, which is then treated with a suitable carbonyl-activating agent such as dicyclohexylcarbodiimide under cyclization conditions to form an acyl imine. The acyl imine is then reacted with a simple conjugated diene to achieve cycloaddition.

Still further compounds within Formula I can be prepared by contacting an α-halotoluic acid with at least two equivalents of an alkali salt of a lower alcohol according to the Williamson ether synthesis to produce an ether linkage. The resulting alkoxymethylbenzoic acid is activated with carbonyldiimidazole, thionyl chloride, dicyclohexylcarbodiimide, or any other suitable activating agent, and reacted with a suitable amine to achieve a carboxamide linkage.

In an alternate to the scheme of the preceding paragraph, a formyl-substituted aromatic carboxamide is prepared by activation of an appropriate starting acid with a tertiary amine (for example, triethyl amine) plus an acid chloride (for example, pivaloyl chloride) to produce a mixed anhydride for coupling to a suitable amine. The formyl group is then reduced to an alcohol by a suitable reducing agent such as sodium borohydride. The alcohol is then converted to a leaving group which is replaceable by the alkali salt of an alcohol. The leaving group can be generated by reagents such as thionyl chloride, thionyl bromide, mineral acids such as hydrochloric, hydrobromic or hydroiodic acids, or the combined action of a tertiary amine plus either a suitable sulfonic anhydride or sulfonyl halide. Alternatively, the alcohol can be activated by removing the proton. This is achieved by the action of a strong base such as sodium hydride in an aprotic solvent such as dimethylformamide. The resulting alkoxide is then reacted with a suitable alkyl halide or other alkyl compound with a suitable leaving group to produce the desired ether linkage.

Fused ring structures such as those in which R³ and one of the R⁸'s of Formula I are combined to form a single linking group bridging the 2 and 3' carbon atoms can be synthesized in the following manner. The carboxyl group of an appropriately substituted salicylic acid is activated with carbonyldiimidazole in dichloromethane, chloroform, tetrahydrofuran, or other anhydrous solvent. An aminoalkylacetal such as H₂N(CH₂)₃CH(OCH₂CH₃)₂ is then added. The resulting amide is treated with an aryl or alkyl sulfonic acid, trifluoroacetic acid, or other strong acid, in a solvent of low basicity such as chloroform or dichloromethane, to cleave the acetal and cyclize the intermediate aldehyde with the amide nitrogen and the phenolic oxygen.

In all of these reaction schemes, the methods and reaction conditions for each of the individual reactions are well within the routine skill of, and will be readily apparent to, the synthesis chemist.

### 2. Screening of compounds

A number of compounds belonging to the above-described genus have been shown to up-modulate glutamatergic transmission by augmenting ligand-AMPA receptor complex-activated ion gating. Staubli, U. et al., 1994a, Proc. Nat. Acad. Sci. U.S.A., 91:777-781; Staubli, U. et al., 1994b, Proc. Nat. Acad. Sci. U. S.A. , 91:11158-11162; Arai, A. et al., 1994, Brain Res., 638:343-346; Granger, R. et al., 1993, Synapse, 15:326-329. These compounds rapidly cross the blood-brain barrier (Staubli, U. *et al.,* 1994b) and increase ESPSs in freely moving rats (Staubli, U. *et al.,* 1994a). Animal experiments indicate that these centrally active modulators improve memory in both rat (Granger, R. *et al.,* 1993; Staubli, U. *et al.,* 1994a) and human models (Lynch et al., 1996, Internal. Clinical Psychopharmacology 11:13; Ingvar *et al.,* submitted to Science).

Once prepared, the compounds used in the invention can be screened for their ability to amplify (upmodulate) the activity of the natural stimulators of AMPA receptors, particularly by amplifying excitatory synaptic responses. A variety of accepted tests can be used to determine whether a given compound is an upmodulator of the AMPA receptor. The primary assay is measurement of the enlargement of the excitatory postsynaptic potential (EPSP) in *in vitro* brain slices, such as rat hippocampal brain slices.

In experiments of this kind, slices of hippocampus from a mammal, such as rat, are prepared and maintained in an interface chamber using conventional methods. Field EPSPs are recorded in the stratum radiatum of region CA1b and elicited by single stimulation pulses delivered once per 20 seconds to a bipolar electrode positioned in the Schaffer-commissural projections (see, Granger, R. et al., Synapse, 15:326-329 1993; Staubli, U. et al., 1994a, Proc. Nat. Acad. Sci., 91:777-781; and Staubli, V. et al., 1994b, Proc. Nat. Acad. Sci., 91:11158-11162; Arai, A. et al., 1994, Brain Res., 638:343-346; Arai, A. *et al.,* "Effects of a centrally active drug on AMPA receptor kinetics, submitted). The wave form of a normal EPSP is composed of an AMPA component, which has a relatively rapid rise time in the depolarizing direction (-5-10 msec) and which decays within -20 msec.;an NMDA component (slow -30-40 msec rise time and slow -40-70 msec decay) (the NMDA portion will not appear in normal or artificial CSF (cerebro-spinal fluid) media, due to the voltage requirement for NMDA receptor channel activation, but in low magnesium media, an NMDA component may appear; a GABA (gamma-aminobutyric acid) component in the opposite (hyperpolarizing) direction as the glutamatergic (AMPA and NMDA) components, exhibiting a time course with a rise time of ~10-20 msec and very slow decay (~50-100 msec or more).

The different components can be separately measured to assay the effect of a putative AMPA receptor enhancing agent. This is accomplished by adding agents that block the unwanted components, so that the detectable responses are essentially only AMPA responses. For example, to measure AMPA responses, an NMDA receptor blocker (*e.g*., AP-5 or other NMDA blockers known in the art) and/or a GABA blocker (*e.g*., picrotoxin or other GABA blockers known in the art) are added to the slice. To prevent epileptiform activity in the GABA-blocked slices, known agents such as tetrodotoxin may be used.

AMPA upmodulators useful in the present invention are substances that cause an increased ion flux through the AMPA receptor complex channels in response to glutamatergic stimulation. Increased ion flux is typically measured as one or more of the following non-limiting parameters: at least a 10% increase in decay time, amplitude of the waveform and/or the area under the curve of the waveform and/or a decrease of at least 10% in rise time of the waveform, for example in preparations treated to block NMDA and GABA components. The increase or decrease is preferably at least 25-50%; most preferably it is at least 100%. How the increased ion flux is accomplished (*e.g*., increased amplitude or increased decay time) is of secondary importance; upmodulation is reflective of increased ion fluxes through the AMPA channels, however achieved.

An additional and more detailed assay is that of excised patches, *i.e*., membrane patches excised from cultured hippocampal slices; methods are described in Arai *et al.,* 1994. Outside-out patches are obtained from pyramidal hippocampal neurons and transferred to a recording chamber. Glutamate pulses are applied and data are collected with a patch clamp amplifier and digitized (Arai *et al.,* 1994). Because no GABA is applied to the patch, GABAergic currents will not be elicited. Any NMDA currents can be blocked as above (*e.g*., with AP-5).

The central action of a drug can be verified by measurement of field EPSPs in behaving animals (*see*, Staubli *et al.,* 1994a) and time course of biodistribution can be ascertained via injection and subsequent quantitation of drug levels in various tissue samples. Quantitation can be accomplished by methods known to those skilled in the art and will vary depending on the chemical nature of the drug.

### C. Other Compounds (not the invention)

The above described genus and species of compounds represent merely one example of glutamatergic compounds that may be used to treat sexual dysfunctions. Other compounds may be administered that enhance the stimulation of α-amino-3-hydroxy-5-methylisoxazole-4-propionic acid ("AMPA") receptors in a subject, said enhancement being sufficient to diminish the symptoms of sexual dysfunction. Examples of other such AMPA-selective compounds include 7-chloro-3-methyl-3-4-dihydro-2H-1,2,4 benzothiadiazine S,S, dioxide, as described in Zivkovic et al., 1995, J. Pharmacol. Exp. Therap., 272:300-309; Thompson et al., 1995, Proc. Nat. Acad. Sci. USA, 92:7667-7671.

### D. Subject selection

Subjects contemplated for treatment with medicament in accordance with this invention include humans, laboratory animals, and domestic animals. It is generally understood that pharmacological treatments (*e.g*., administration of testosterone) influences sexual behavior in these animals.

The compounds and compositions described above are generally most useful to treat sexual dysfunctions in subjects having no demonstrable organic cause for the disorder. Conditions responsive to glutamatergic treatment include cases of reduced sexual drive, arousal or desire, age-associated hyposexuality unaccompanied by other physiological indications or by difficulties reaching orgasm.

Although certain conditions (*e.g*., some that arise from surgical interventions (*e.g*., for prostate conditions) and those diagnosed as vascular impotence (*e.g*., anatomic inability to achieve erection with intact psychological desire) are less likely to respond, the compounds used in the present invention are useful to treat individuals afflicted with physical disorders often also have psychogenic inhibitions that exacerbate the physical problem. Treatment of the psychogenic component ameliorates the reduced sexual drive, arousal or desire, age-associated hyposexuality, and facilitates treatment of the physical component. Thus, even in cases of physical sexual dysfunction, combinations of hormone and physical treatments may be indicated.

### E. Administration of compounds

The compounds used in this invention can be incorporated into a variety of formulations for therapeutic administration. Examples are capsules, tablets, syrups, suppositories, and various injectable forms. Administration of the compounds can be achieved in various ways, including oral, bucal, rectal, parenteral, intraperitoneal, intradermal, transdermal, etc., administration.

Preferred formulations of the compounds are oral preparations, particularly capsules or tablets containing each from about 1 milligram up to about 100 milligrams of active ingredient.

### F. Dosage

The above described compounds and/or compositions are administered at a dosage that diminishes the symptoms of sexual dysfunction in subjects suffering from a sexual dysfunction while at the same time minimizing any side-effects. It is contemplated that the composition will be obtained and used under the guidance of a physician.

Typical dosages for systemic administration range from 0.1 to 1000 milligrams per kg weight of subject per administration. A typical dosage may be one 10-50 mg tablet taken once a day, or one time-release capsule or tablet taken once a day and containing a proportionally higher content of active ingredient. The time-release effect may be obtained by capsule materials that dissolve at different pH values, by capsules that release slowly by osmotic pressure, or by any other known means of controlled release.

Dose levels can vary as a function of the specific compound, the severity of the symptoms, and the susceptibility of the subject to side effects. Some of the specific compounds that stimulate glutamatergic receptors are more potent than others. Preferred dosages for a given compound are readily determinable by those of skill in the art by a variety of means. A preferred means is to measure the physiological potency of a given compound that is a candidate for administration, by the method of Davis et al. (1996), submitted to Behavioral Neuroscience. Briefly, excised patches and excitatory synaptic responses are measured in the presence of different concentrations of test compounds, and the differences in dosage response potency are recorded and compared. Davis *et al.* found that one specific compound designated BDP-20 was about ten-fold more potent than another designated BDP-12 in a variety of behavioral (exploratory activity, speed of performance) and physical (excised patches and excitatory synaptic responses) tests. The relative physiological potency was an accurate measure of their behavioral potency. Thus, excised patches and excitatory synaptic responses may be used to gauge the relative physiological (and behavioral) potency of a given compound with regard to a known standard.

Preferred glutamatergic compounds for the treatment of sexual dysfunctions may have a half-life measured from less than 10 minutes to more than 2 hours. In some embodiments, the compound preferably has a rapid onset and short elimination half-life (≤ 90 min.).

### EXAMPLES

The following examples are submitted for illustrative purposes.

### A. Preparation of Compound D1 (7-chloro-5-ethyl-3-methyl-3,4-dihydro-2H-1,2,4 benzothiadiazine S,S-dioxide)

### 1. Preparation of 5-chloro-3-ethyl-2-aminobenzenesulfonamide (D)

To a solution of 3-ethyl-2-aminobenzenesulfonamide (0.5043g, 2.52 mmol) in 10 mL acetonitrile was added *N*-chlorosuccinimide (0.3163g, 2.37 mmol). The mixture was stirred at reflux for 12.5 h, cooled, filtered, and concentrated *in vacuo.* Reaction was incomplete by ¹H NMR; the purple solids were redissolved in acetonitrile and additional NCS (0.1019g, 0.76 mmol) was added. The mixture was refluxed for 15 minutes and worked up as before. Column chromatography on the mixture of succinimide and *D* (40% ethyl acetate:hexanes) gave pure *D* (0.5224g, 94%), m.p. 134-135°C. ¹H NMR (300 MHz, DMSO): δ 7.45 (3H, s), 7.19 (1H, d, J = 2.1 Hz), 5.76 (2H, s), 2.53 (2H, q, J = 7.3 Hz), 1.14 (3H, i, J = 7.3 HZ). ¹³C NMR (300 MHz, DMSO): δ 141.9, 131.8, 131.2, 125.4, 124.8, 118.1, 23.4, 12.7. FT-IR (KBr): 3457.8, 3427.9, 3358.4, 3268.9, 3080.1, 2970.8, 2364.5, 2334.7, 1630.6. 1556.1, 1541.2. 1451.7. 1312.6. 1188.3, 1148.6, 905.1, 875.3, 762.2, 611.3 cm⁻¹. HRMS: M⁺ calculated = 234.0230, found = 234.0227.

### 2. Preparation of 7-chloro-5-ethyl-3-methyl-3,4-dihydro-2H-1,2,4 benzothiadiazine S,S-dioxide (D1)

To a 25 mL RB flask was added 5-chloro-3-ethyl-2-aminobenzenesulfonamide *D* (0.4074 g, 1.74 mmol), 8.5 mL acetonitrile, and 3Å molecular sieves. The stirring solution was cooled to 0°C and acetaldehyde (0.15 mL, 2.68 mmol) and (±) 10-camphor sulphonic acid (catalytic) were added. After 1 h at 0°C, the reaction was filtered through Celite and solvent was removed to give crude *D1.* The light brown solid was dissolved in EtOAc, passed through silica, and concentrated to give pure *D1* (0.4426 g, 98%), m.p. 181-183°C. ¹H NMR (300 MHz, DMSO): δ 7.70 (1H, d, J=11.8 Hz), 7.35 (1H, d, J = 2.2 Hz), 7.21 (1H, d, J = 2.2 Hz), 6.24 (1H, s), 4.80 (1H, m), 2.52 (2H, m), 1.48 (3H, d, 7.1 Hz), 1.13 (3H, t, J=7.4 Hz). ¹³C NMR (500 MHz, DMSO): δ 140.2, 132.4, 131.1, 122.2, 120.5, 120.0, 62.2, 23.2, 20.1, 12.7. FT-IR (KBr): 3405.9, 3232.5, 3001.5, 2942.7, 2884.0, 1490.5, 1438.5, 1309.6, 1282.4, 1160.2, 1140.1, 864,5, 743.8, 718.0, 567.5 cm⁻¹. HRMS: M⁺ calculated = 260.0386, found = 260.0386.

Those of skill in the art will readily appreciate that the foregoing protocol can be used, with only minor modifications, to prepare the other compounds of the present invention.

### B. Administration of AMPA-kines to rats

Rats and mice have traditionally been the quintessential test animals for developing animal models for treating disease. A large body of studies indicates that hormones and treatments that modulate sexual behavior in rodents also do so in other mammals, humans included.

In the male rat, "age-related sexual dysfunctions" or deficits of sexual arousal/motivation occur as early as one year of age. This form of sexual deficit is apparently not reversible by administration of exogenous testosterone (Gray *et al.,* 1981).

Although early studies indicated the possibility of differences in plasma levels of testosterone in old mating versus non-mating males, subsequent batteries of experiments have failed to replicate these initial findings, and indicate that decline in testosterone is unlikely to be the primary cause of sexual dysfunction with increasing age (Smith et *al.,* 1992). It seems probable that the major alterations responsible for the age-related changes in behavior reside in the CNS, and these are likely to be non-hormone dependent.

Central glumatergic enhancement has been shown to have positive effects on age-related declines in learning (Granger *et al.,* 1996). We undertook experiments to test whether central glutamatergic enhancement would also affect age-related declines in sexual performance.

Two of the primary characteristics of sexual deficit in aged rats are those of delay. Both latency to intromit and latency to ejaculate are significantly lengthened with increasing age (Smith and Davidson, 1990; Smith *et al.,* 1992).

In the present experiments, aged (16-18 months) male Long-Evans rats (n = 9) were paired with receptive females for 30-minute periods once a week for two months, 30 minutes after receiving injections of either saline or a centrally-active glutamatergic enhancer. On the days that they received the drug, animals exhibited significantly decreased latencies to intromit (IL) and to ejaculate (EL) as compared with their performance on control days. Figure 1 shows the means and standard errors for IL (p < 0.05 by the Wilcoxon test) and EL (p < 0.03 by the Wilcoxon test) on control (white) and drug (striped) days for a set of nine animals.

The observed changes in IL and EL with the glutamatergic enhancer are thus consistent with amelioration of age-related sexual impairment, suggesting that central glutamatergic enhancement may serve as a novel treatment for this condition.

### C. Administration to humans

As mentioned above, not all sexual dysfunctions are treated with the compounds used herein. Thus, a first step in treating humans is generally determining which individuals have dysfunctions which are likely to respond (*e.g*., neurogenic, psychogenic or age-related sexual dysfunctions), and which will not.

Determination whether a subject is suffering from a sexual dysfunction that is substantially neurogenic or psychogenic in origin is made by a person skilled in the art using a number of readily available diagnostic procedures. In general, the presence of typical DSMIII sexual dysfunctions in humans can be ascertained via questionnaires or interviews. Spontaneous self-identification by individuals of a low or comparatively diminished desire or ability to function sexually and to experience sexual pleasure is one criterion for selecting individuals to be treated. Few laboratory tests are required for the routine evaluation of common sexual complaints (Kligman, 1991). Impotence which is a side-effect of drugs such as beta-blockers or alcohol is deemed to be neurogenic impotence in the present specification.

Although the psychosexual history of the subject may be the only criterion used to select candidates for treatment, it may be desirable to also rule out purely physical conditions that are not treatable with the compounds used in the invention should be ruled out.

### 1. Diagnosis of dysfunctions that are substantially only neurogenic or psychogenic In origin

A subject suffering from impotence can be given a physical examination with particular attention to possible penile and scrotal pathology precluding an erection sufficient for vaginal penetration. Such tests include determination of the penobrachial blood pressure index (PBPI), Doppler investigation of the penile arteries, and a papaverine test. The subject may further be tested for signs of penile venous leakage or atherosclerosis.

The PBPI is the penile systolic blood pressure divided by the systolic blood pressure determined at one of the arms. These blood pressures can be determined by any numerous standard techniques. Thus, the penile systolic blood pressure can be determined by placing around the base of the free part of the penis in the flaccid state, an inflatable cuff, which is capable of being used to apply variable pressure, readable from a gauge, to an object around which the cuff is placed, localizing the penile arteries with a Doppler ultrasound probe (*e.g*., and 8 MHz probe, such as the Mini Doplex D500 TM available from Huntleigh Technology, Luton, United Kingdom), and then inflating and deflating the cuff and ascertaining the pressure at which the Doppler sound reappears. The pressure at which the Doppler sound reappears is the penile systolic blood pressure. A male's penile blood pressure is regarded as normal if his PBPI is > 0.80.

With regard to Doppler investigation, each of the two penile cavernous arteries is investigated distal to the aforementioned cuff using Doppler ultrasound. The function of each of the two arteries is assessed by Doppler ultrasound using an arbitrary scale of 0, 1, 2 or 3, where 0 means that the function is so deficient ultrasound using an arbitrary scale of 0, 1, 2 or 3, where 0 means that the function is so deficient that the artery cannot be located and 3 means that the artery is well enough that maximal Doppler sound is observed.

In the papaverine test, a tourniquet is placed at the base of the free part of the penis and tightened and then, with the patient seated, 30 mg of papaverine in 1 ml of a physiologically acceptable fluid such as physiological saline or phosphate-buffered saline is injected into the penile cavernous body. (In persons suspected of having impotence due to a suprasacral nerve lesion or a psychogenic dysfunction, only 15 mg of papaverine is administered, because of the high incidence of papaverine-induced priapism in such cases.) Five minutes after the injection, the tourniquet is removed and an ultrasound Doppler investigation of the penile cavernous arteries is carried out as described above. The function of the arteries is regarded as normal if both of them score a 3 on the arbitrary scale. After the Doppler investigation, penile vibration, at about a 4 Hz with an amplitude of about 1.2 mm (carried out with, *e.g*., a Vibrector^{™} from Multicept, Gentofte, Denmark) is performed out for five to ten minutes and then erectile response is evaluated. Erectile response is classified as full rigidity, if the angle between the penis and the legs in the standing position is > 90°, and tumescence or no response if the angle is less than or equal to 45°. The papaverine employed in the "papaverine test" described above could be replace with VIP (approximately 50 g).

An impotent male who does not have an anatomical deficiency that would preclude having an erection sufficient for vaginal penetration, who has a PBPI > 0.80, who scores of 2 or 3 in Doppler ultrasound investigations of both of the cavernous arteries of the penis after papaverine injection as described above, and who has a fully rigid erection after papaverine injection and vibration as described above, is suffering from neurogenic or psychogenic dysfunction.

Generally, a male with a PBPI less than about 0.60, with scores of 0 in Doppler investigations of both penile cavernous arteries (after papaverine injection as described above), and with a less than fully rigid erection after papaverine injection and vibration will have impotence caused by "untreatable" atherosclerosis.

Whether a subject suffers from impotence that is untreatable because of venous leakage may be determined by a number of methods. One method is cavernosometry, optionally supplemented with cavernosography. See, *e.g*., Delcour et al. (1986) Radiology 161:799; Porst et al. (1987) J. Urol. 137: 1163; Lue et al. (1987) J. Urol. 37: 829. Cavernosometry can be performed using, both before and after intracavernosal injection of 60 mg of papaverine (in 1 ml of physiological saline), infusion of physiological saline through a 19-gauge needle into one corpus cavernosum with a 21-gauge needle inserted into the other corpus cavernosum for measurement of intracorporal pressure (which is recorded on a plotter). The infusion rates needed to induce and maintain an erection are measure. If the infusion rate needed to maintain an erection is greater than 50 ml/min before administration of the papaverine and greater than 15 ml/min after administration of the papaverine, untreatable venous leakage is present. As long as an erection can be achieved at some flow rate less than about 100 ml/min before injection of the papaverine and less than about 50 ml/min after the injection of papaverine, it might be possible, using cavernosography, to locate the venous lesion associated with the leakage, and thereby confirm the diagnosis based on cavernosometry and provide information for possible surgical correction for the leakage. In the cavernosography, the penis is X-rayed, both before and after intracavernosal injection of 60 mg papaverine in 1 ml of physiological saline, while infusing contrast medium into the corpus cavernosum (*e.g*., through a 19-gauge needle) at a flow rate that maintains an erection during the x-raying. Numerous contrast media suitable for the procedure are available in the art; these are typically aqueous solutions of iodinated compounds that provide between about 180 mg/ml and about 360 mg/ml of iodine. Examples are Omnipaque 240™, which is a solution of iohexol providing 240 mg/ml of iodine sold by Winthrop Pharmaceuticals, New York, N.Y., USA, and lopamiro (Registered TM), which is a solution of iopamidol providing 300 mg/ml iodine sold by Astra Meditec, Goteborg, Sweden. Typically 50-100 ml of the contrast medium will be employed for each x-ray (i.e., before and then after the injection of papaverine). In the cavernosometry and cavernosography, 30 mg papaverine (in 1 ml physiological saline) coupled with stimulation by vibration can be employed in place of 60 mg papaverine (in 1 ml physiological saline).

### 2. Assessing progress of the treatment

The success of treatment is measured by monitoring and recording the nature, number, frequency and duration of instances of sexual stimulation and sexual behavior. The sexual behavior to be monitored can comprise sexual relations between the patient and another person or can involve sexual behaviors that do not involve another person. Sexual responsiveness may be induced and gauged by: touching or erotically manipulating erogenous areas of the genital organs or other erogenous parts of the body; providing visual stimulation, as with a sexually explicit film or other form of sexually stimulating show or display; or providing vibratory stimulation to the penis (for example, by resting the penis on the table of a vibrating apparatus such as that of a Vibrector system (Multicept, Genofte, Denmark), at between about 30 Hz and about 100 Hz with an amplitude of about 1 to about 5 mm), alone or in combination. A preferred method of sexual stimulation includes providing visual stimulation, as with a sexually explicit film, simultaneously with vibratory stimulation of the penis, as with a Vibrector system set to between about 30 Hz and about 60 Hz (usually about 50 Hz) in frequency and between about 1 mm and about 2.5 mm (usually about 2.2 mm) in amplitude.

The progress of the treatment is monitored by having the patient record instances and frequency of sexual behavior or of subjective sexual arousal, for example by keeping a diary. In addition, the supervising physician will maintain a log of treatments and observations. In combination, the diary and log will allow for a comparison of instances and frequency of sexual behavior or of subjective sexual arousal before and in response to treatment.

### D. Kits

In addition, the present invention provides for kits with unit doses of AMPA up-modulating drugs either in oral or injectable doses. In addition to the containers containing the unit doses will be a informational package insert describing the use and attendant benefits of the drugs in treating neurodegenerative pathologies not significantly affecting memory or learning. Preferred compounds and unit doses are those described herein above.

## Claims

1. A compound of the formula 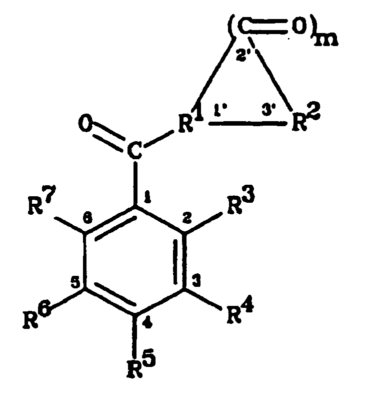 wherein
R¹ is N or CH;
m is 0 or 1;
R² is (CHR⁸)ₙ₋ₘ or Cₙ₋ₘHR⁸₂₍ₙ₋ₘ₎₋₃, in which n is 4, 5, 6, or 7, the R⁸'s in any single compound being the same or different, each R⁸ being a member selected from the group consisting of H and C₁-C₆ alkyl;
R³ is a member selected from the group consisting of H, C₁-C₆ alkyl, and C₁-C₆ alkoxy;
R⁶ is H;
R⁴ and R⁵ are combined to form a member selected from the group consisting of 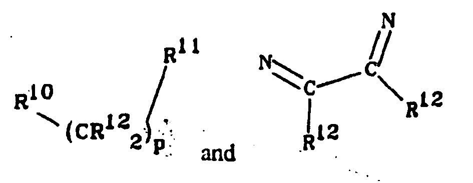
in which:
R¹⁰ and R¹¹ are O;
R¹² is a member selected from the group consisting of H and CH₃;
p is 1 or 2; and
R⁷ is a member selected from the group consisting of H, C₁-C₆ alkyl, and C₁-C₆ alkoxy;
wherein said compound is capable of upmodulating the response of α-amino-3-hydroxy-5-methyl-isoxazole-4-propionic acid ("AMPA") receptors to natural ligand binding,
for use in the treatment of sexual dysfunction, wherein the sexual dysfunction is neurogenic, psychogenic or age-related.

2. A compound as claimed in claim 1, wherein the sexual dysfunction is male sexual dysfunction.

3. A compound as claimed in claim 1 or 2, wherein the sexual dysfunction is male erectile disorder.

4. A compound as claimed in claim 1 or 2, wherein the sexual dysfunction is inability to ejaculate.

5. A compound as claimed in claim 1 or 2, wherein the sexual dysfunction is inability to experience an orgasm.

6. A compound as claimed in any preceding claim for oral administration.

7. A compound as claimed in any preceding claim for administration by injection.

## Patentansprüche

1. Eine Verbindung mit der Formel 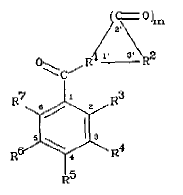 worin;
R¹ N oder CH ist;
m 0 oder 1 ist;
R²(CHR⁸)ₙ₋ₘ oder Cₙ₋ₘHR⁸₂₍ₙ₋ₘ₎₋₃ ist, wobei n 4, 5, 6 oder 7 ist, R⁸ in jeder einzelnen Verbindung gleich oder unterschiedlich ist und jedes R⁸ ein Mitglied ausgewählt aus der Gruppe bestehend aus H und C₁-C₆-Alkyl ist;
R³ ein Mitglied ausgewählt aus der Gruppe bestehend aus H, C₁-C₆-Alkyl und C₁-C₆-Alkyloxy ist;
R⁶ H ist;
R⁴ und R³ zusammengesetzt werden, um ein Mitglied ausgewählt, aus der Gruppe bestehend aus 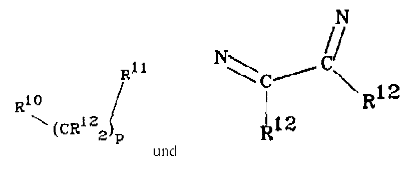
zu bilden wobei:
R¹⁰ und R¹¹ O sind:
R¹² ein Mitglied ausgewählt aus der Gruppe bestehend aus H und CH₃ ist; p 1 oder 2 ist und
R⁷ ein Mitglied ausgewählt aus der Gruppe bestehend aus H, C₁-C₆-Alkyl und C₁-C₆-Alkyloxy ist,
wobei die Verbindung geeignet ist, die Antwort von Rezeptoren für α-Amino-3-Hydroxy-5-Methyl-Isoxazol-4-Propionsäure ("AMPA") zur natürlichen Ligandenbindung aufzumodulieren,
zur Verwendung in der Behandlung von sexuellen Dysfunktionen, wobei die sexuelle Dysfunktion neurogen, psychogen oder altersbedingt ist.

2. Eine Verbindung gemäß Anspruch 1, wobei die sexuelle Dysfunktion eine sexuelle Dysfunktion eines Mannes ist.

3. Eine Verbindung gemäß Anspruch 1 oder 2, wobei die sexuelle Dysfunktion eine Ercktionsstörung eines Mannes ist.

4. Eine Verbindung gemäß Anspruch 1 oder 2, wobei die sexuelle Dysfunktion die Unfähigkeit zur Ejakulation ist.

5. Eine Verbindung gemäß Anspruch 1 oder 2, wobei die sexuelle Dysfunktion die Unfähigkeit ist, einen Orgasmus zu erlangen.

6. Eine Verbindung gemäß einem der vorhergehenden Ansprüche zur oralen Verabreichung.

7. Eine Verbindung gemäß einem der vorhergehenden Ansprüche zur Verabreichung durch Injektion.

## Revendications

1. Composé de formule 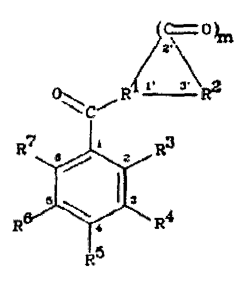 dans laquelle
R¹ est N ou CH ;
m vaut 0 ou 1;
R² est (CHR⁸)_{n m} ou Cₙ₋ₘHR⁸_{z(n m)-3} où n vaut 4, 5, 6 ou 7, les R⁸ dans tout composé individuel étant identiques ou différents, chaque R⁸ étant un élément choisi dans le groupe consistant en II et un groupe alkyle en C₁ à C₆ ;
R³ est un élément choisi dans le groupe consistant, en H, un groupe alkyle en C₁ à C₆ et alcoxy en C₁ à C₆ R⁶ est. H ;
R⁴ et R⁵ sont combinés pour former un élément choisi dans le groupe consistant en 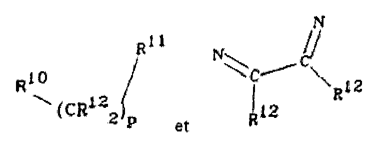
dans lesquels :
R¹⁰ et R¹¹ sont O;
R¹² est un élément choisi dans le groupe consistant en H et CH₃ ;
P vaut 1 ou 2 ; et
R⁷ est un élément choisi dans le groupe consistant en H, un groupe alkyle en C₁ à C₆, et alcoxy en C₁ à C₆;
où ledit composé est capable de moduler à la hausse la réponse de récepteurs d'acide α-amino-3-hydroxy-5-méthyl-isoxazole-4-propionique (« AMPA ») à une liaison à un ligand naturel,
à utiliser dans le traitement d'un dysfonctionnement sexuel, où le dysfonctionnement sexuel est neurogène, psychogène ou lié à l'âge.

2. Composé selon la revendication 1, dans lequel le dysfonctionnement sexuel est un dysfonctionnement sexuel masculin.

3. Composé selon la revendication 1 ou 2, dans lequel le dysfonctionnement sexuel est un dysfonctionnement érectile.

4. Composé selon la revendication 1 ou 2, dans lequel le dysfonctionnement sexuel est l'incapacité à éjacule.

5. Composé selon la revendication 1 ou 2, dans lequel le dysfonctionnement sexuel est l'incapacité à connaître un orgasme.

6. Composé selon l'une quelconque des revendications précédentes, pour administration orale.

7. Composé selon l'une quelconque des revendications précédentes, pour administration par injection.
